(19) Europäisches Patentamt
European Patent Office
Office européen des brevets

(11) Publication number: **0 292 596 B1**

# (12) EUROPEAN PATENT SPECIFICATION

(45) Date of publication of patent specification: **30.09.92** (51) Int. Cl.5: **A61N 1/05**, H01R 43/00

(21) Application number: **87107760.8**

(22) Date of filing: **27.05.87**

## (54) Method of electrically connecting conductors and electrodes of an implantable electrode lead.

(43) Date of publication of application:
**30.11.88 Bulletin 88/48**

(45) Publication of the grant of the patent:
**30.09.92 Bulletin 92/40**

(84) Designated Contracting States:
**DE FR GB IT NL SE**

(56) References cited:
**FR-A- 2 245 103**
**US-A- 3 701 877**
**US-A- 4 253 233**

(73) Proprietor: **Siemens Elema AB**
**Röntgenvägen 2**
**S-171 95 Solna 1(SE)**
(84) Designated Contracting States:
**SE**

(73) Proprietor: **SIEMENS AKTIENGESELLSCHAFT**
**Wittelsbacherplatz 2**
**W-8000 München 2(DE)**
(84) Designated Contracting States:
**DE FR GB IT NL**

(72) Inventor: **Hirschberg, Jakub**
**Aprilvägen 3**
**S-183 44 Täby(SE)**

EP 0 292 596 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method of electrically connecting conductors and electrodes of an implantable lead.

A lead for biomedical stimulation comprises a lead body and one or more exposed electrodes for making electrical contact with body tissues. The lead body essentially includes one or more conductors electrically connected to the electrodes, and one or more insulating materials enclosing the conductors and also forming a lead casing. Generally, the casing is made of a flexible, body compatible material such as silicone rubber or the like. Between each electrode and the lead body there is a juncture, which is critical to the proper functioning of the lead. The juncture must withstand body fluid penetration and, of interest in this application, ensure the electrical connection between the electrode and the related conductor.

There are various prior art methods of making such electrical connections.

Thus, e.g. US-A-4 381 014 teaches simultaneous connecting and sealing of a juncture by welding an axially extending supplementary wire to several turns of a single-pole, helix-formed conductor at predetermined electrode locations before the conductor is encased in the insulating sheath. Subsequently, the electrode is swaged to cause the wire to penetrate the insulating sheath and establish electrical connection of the conductor and the electrode. Simultaneously, the crimped (swaged) electrode seals the juncture at the ends of the electrode.

An example of a connection in a bi-polar lead is given in US-A-3 769 984. The lead in that document comprises two parallel, longitudinally extending non-helical wire conductors in an extruded lead body. The conductors are connected to a pair of axially separated electrodes at the lead distal end. The axial separation between the electrodes corresponds to the axial separation between the distal ends of the conductors, which ends are brought out from the lead body through bores and then connected to the electrodes by e.g. being clamped between a crimp ring and the electrode.

In these exemples, with one or two conductors in the lead body, conductor selection at a desired electrode location is unproblematic with respect to the connection method. In contrast, modern tubing leads, as disclosed in e.g. US-A-4 484 586, require the electrodes to be attached to a tubing lead body structure comprising several, individually insulated, helically wound conductors. The axial separation between the turns of different conductors in the helix is very small, e.g. about 0,2 mm. From this bundle, the conductor to be connected to a certain electrode should be quickly and reliably selected, made free from insulation and adapted for connection at a desired electrode location.

From FR-A-2 245 103 there is known a method for establishing an electric connection to a selected wire of a cable comprising several insulated wires enclosed by an outer insulating sheath. First, a section of the outer insulating sheath is removed, then a conducting element is inserted where the sheath is removed until it is close to the insulated wires, thereafter a conductive liquid is introduced into the space between the insulated wires and the conducting element, an increasing voltage is applied between the selected wire and the conducting element until dielectric breakdown of the insulation occurs so that the liquid can come into contact with the wire, and finally the conductive liquid is solidified.

It is an object of the present invention to provide an electrode-conductor connecting method which is suitable for, but not exclusively applicable to, a conductor in a lead of the tubing type described in US-A-4 484 586.

The above object is inventively achieved in the method defined in the claims.

In order that the invention may be more readily understood reference will now be made to the accompanying drawings.

Fig. 1 is an axial sectional view of the lead illustrating the puncturing and the high voltage burning steps of the method.

Fig. 2 is an axial sectional view of the lead illustrating the step in the method where the conductor selected for connection is taken out with a curved tool.

Fig. 3 is identical to Fig. 2 except that it illustrates the selected conductor being resistance welded to a supplementary electrode connecting conductor.

Fig. 4 is a perspective view of the lead illustrating how slitted (tubing) rings and crimp rings are applied to the lead. Also, the assembly of a guide coil is illustrated.

Referring now to the figures, in Fig. 1 an embodiment of the invention discloses a multi-pole lead I having helically wound conductors in a four-pole arrangement $2_1$, $2_2$, $2_3$, $2_4$. The conductors may consist of multi-filament (e.g. refined steel) wire, which exhibits a high fatigue strength under repeated bending stress. Preferably, but not necessarily, the conductors could be surrounded by a first insulating material 3 with good electric insulating properties, viz. teflonpolymers teflon is a registered trade mark such as FEP (Fluorinated ethylene propylene) or PTFE (Polytetrafluorethylene). The conductors are embedded in a second insulating material 4 with good body compatibility properties, e.g. silicone rubber. The conductors 2 may be individually identified by e.g. coloured insulation, possibly combined with an X-ray contrast medium.

The connecting method comprises the following steps. The conductor to be connected, say $2_1$, is arranged as one pole in a high voltage circuit 11. The puncturing means, a needle 8 or the like is arranged as the other pole in the circuit and placed at the desired electrode location, but at a distance from the lead body.

By increasing the voltage, an electric arc is initiated between the conductor $2_1$ and the needle 8, and the insulation materials are punctured. As the flash-over takes place only between the poles in the circuit the conductors $2_2$, $2_3$ and $2_4$ and their insulations are left undisturbed even if the conductors form a compact multi-pole helix, and the method does not require the needle 8 to be exactly located over the conductor $2_1$. By maintaining the arc, a hole (12; Fig. 2) is burnt in the insulating materials through which the conductor is made available for the next step in the method.

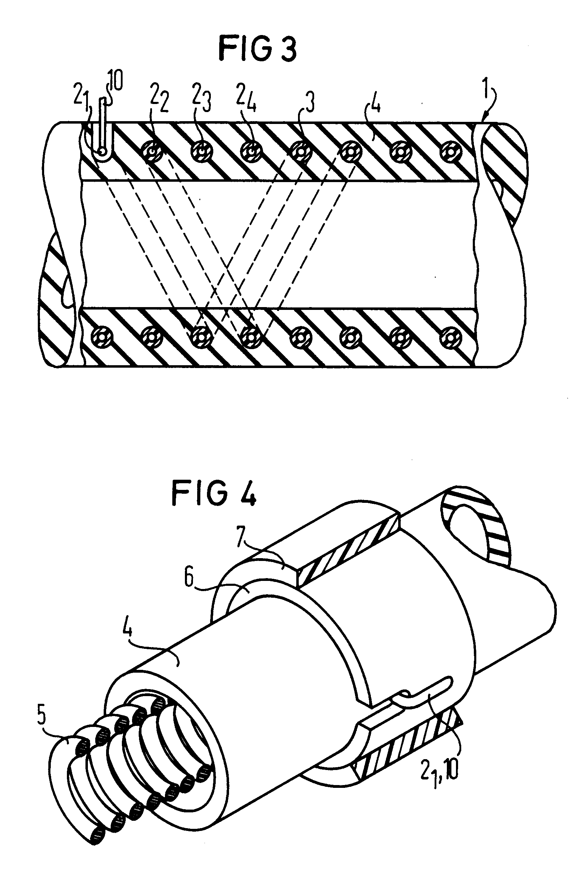

In Fig. 2 the step is illustrated, in which the conductor is brought out from the hole (12) with a curved tool 9. The lower position shows the inserted tool in dashed lines and the upper position shows the tool and the brought-out conductor. Alternatively (Fig.3), a supplementary electrode connecting conductor 10 could be inserted into the hole and connected by resistance welding to the exposed turn of the helix-formed conductor, whereby several, axially separated connections to the same or different electrodes are made possible as the helix-formed conductor is not mecanically destroyed. In either case, a connecting end to be applied to the electrode is formed.

Subsequently (Fig. 4) and in a conventional fashion, slitted (tubing) rings 6 are applied to the lead at the hole location and the connecting end is clamped between the slitted ring and the electrode crimp ring 7. The slitted rings 6 and the crimp rings 7 (Fig. 4) should be of a material with good body compatibility properties such as platinum or the like. Alternately, welding could replace clamping.

Finally and also conventionally the lead is assembled with respect to e.g. guide coil (5), connector details, electrode tip and other metal details, and rubber details (e.g. seals) are molded or glued to the lead. The guide coil 5 conductors may be comprised of MP 35 N, possibly in a multi-filament arrangement.

## Claims

1. A method of electrically connecting an electrode (6,7) to an implantable lead (1) comprising at least one conductor (21-24) to be connected to said electrode and at least one insulating sheath (4), said method comprising the following steps:
   (a) arranging a selected one of said at least one conductor to form one pole in a high voltage circuit (11) in which puncturing means (8) forms the other pole,
   (b) puncturing, at a desired electrode position, said at least one insulating sheath by initiating an electric arc between said two poles,
   (c) burning, by maintaining said arc, the insulation where punctured to form a hole (12) through which the selected conductor is available from the outside,
   (d) providing a connecting end for connection between the conductor and the electrode, and
   (e) connecting the electrode and said connecting end.

2. A method according to claim 1, **characterized in that** the connecting end is formed from the conductor brought out from said hole by means of a tool (9).

3. A method according to claim 1, **characterized in that** the connecting end is formed from a supplementary conductor (10) connected to said lead conductor by resistance welding.

## Patentansprüche

1. Verfahren zum elektrischen Verbinden einer Elektrode (6, 7) mit einer implantierbaren Leitung (1), die mindestens einen Leiter (21-24) zur Verbindung mit der Elektrode sowie mindestens einen Isoliermantel (4) aufweist, wobei das Verfahren folgende Schritte umfaßt:
   (a) Vorsehen eines ausgewählten Leiters als ein Pol in einem Hochspannungs-Stromkreis (11) in dem Punktierungsmittel (8) den anderen Pol bilden,
   (b) Punktieren des mindestens einen Isoliermantels bei einer gewünschten Position für die Elektrode, indem ein elektrischer Bogen zwischen den beiden Polen erzeugt wird,
   (c) Wegbrennen der Isolierung durch Aufrechterhalten des elektrischen Bogens an der Stelle der Punktierung, um ein Loch (12) auszubilden, durch das der ausgewählte Leiter von außen her zugänglich ist,
   (d) Vorsehen eines Verbindungsendes zum Anschließen zwischen dem Leiter und der Elektrode und
   (e) Verbinden der Elektrode und des Verbindungsendes mit einander.

2. Verfahren nach Anspruch 1, **dadurch gekennzeichnet,** daß das Verbindungsende von dem Leiter gebildet wird, indem dieser mittels eines Werkzeugs (9) aus dem Loch herausgeholt wird.

3. Verfahren nach Anspruch 1, **dadurch gekennzeichnet,** daß das Verbindungsende von einem Hilfsleiter (10) gebildet wird, der durch Widerstandsschweißen mit dem Leiter der Leitung verbunden wird.

**Revendications**

1. Un procédé pour connecter électriquement une électrode (6, 7) à un câble implantable (1) comprenant au moins un conducteur ($2_1$-$2_4$) devant être connecté à l'électrode et au moins une gaine isolante (4), ce procédé comprenant les étapes suivantes :
   (a) on fait en sorte qu'un conducteur sélectionné parmi les conducteurs précités forme un pôle dans un circuit de haute tension (11) dans lequel des moyens de perçage (8) forment l'autre pôle,
   (b) on perce, à une position d'électrode désirée, la ou les gaines isolantes, en amorçant un arc électrique entre les deux pôles,
   (c) on brûle, en maintenant l'arc précité, l'isolation à l'endroit auquel elle est percée, pour former un trou (12) à travers lequel le conducteur sélectionné est accessible de l'extérieur,
   (d) on établit une extrémité de connexion, pour la connexion entre le conducteur et l'électrode, et
   (e) on connecte l'électrode et l'extrémité de connexion.

2. Un procédé selon la revendication 1, caractérisé en ce que l'extrémité de connexion est formée à partir du conducteur qui est extrait du trou au moyen d'un outil (9).

3. Un procédé selon la revendication 1, caractérisé en ce que l'extrémité de connexion est formée à partir d'un conducteur supplémentaire (10) qui est connecté au conducteur du câble par soudage par résistance.

FIG 1

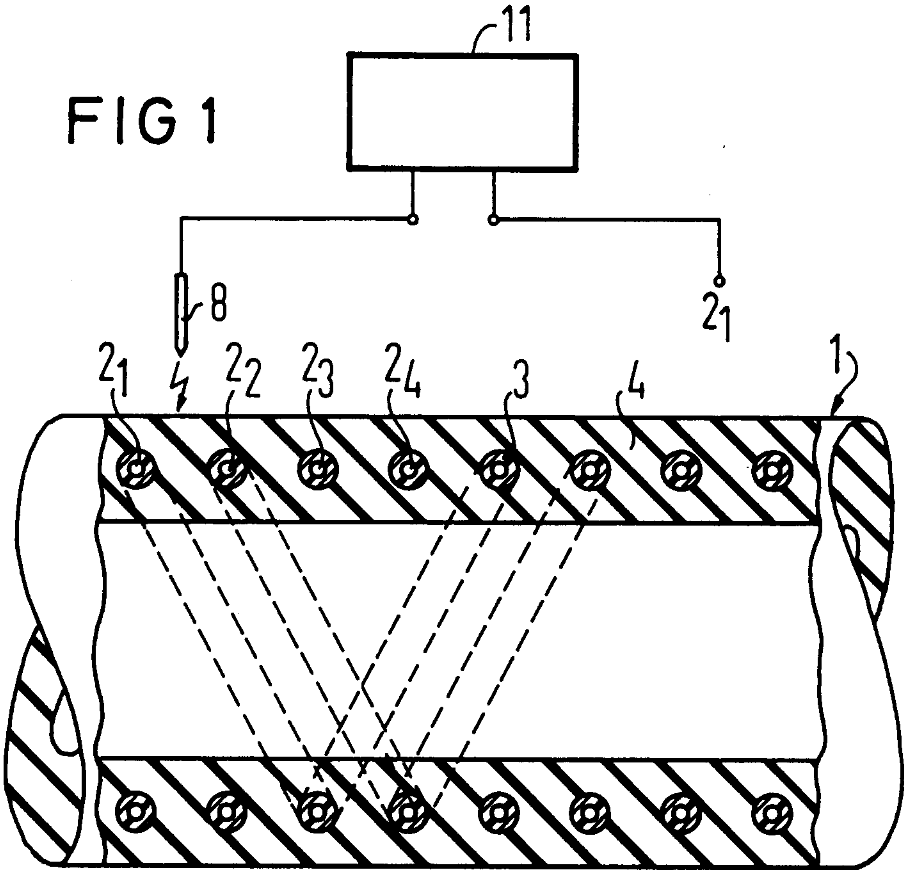


FIG 2

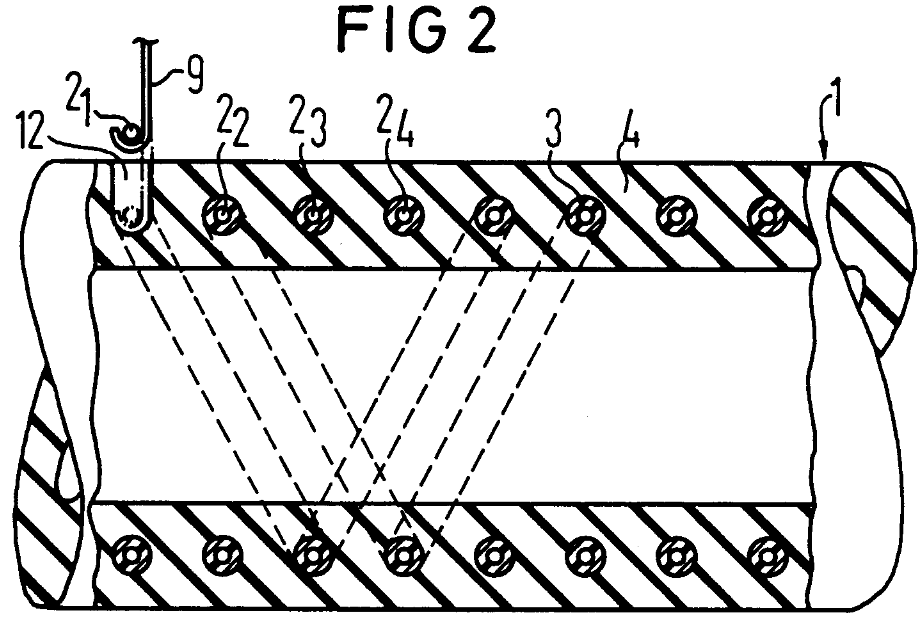

FIG 3

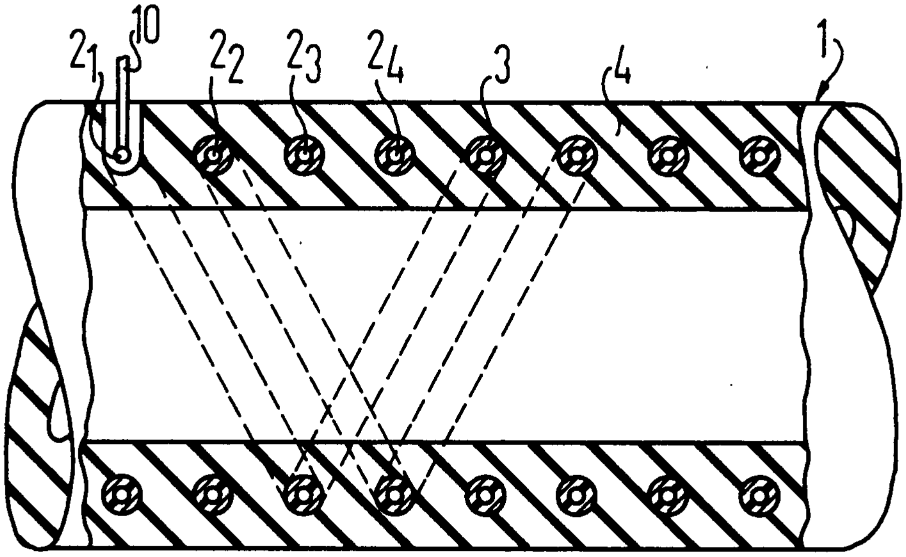


FIG 4